# FASCICULE DE BREVET EUROPEEN

(11) **EP 3 774 075 B1**
(45) Date de publication et mention de la délivrance du brevet: **12.08.2026**
(21) Numéro de dépôt: 19721106.3
(22) Date de dépôt: 28.03.2019
(51) Int. Cl.: B05C 17/005, A61B 17/00, B05C 17/00, A61B 90/00

(54) **EMBOUT ET DISPOSITIF D'APPLICATION ET D'ETALEMENT D'UN PRODUIT DE TYPE LIQUIDE, GEL OU PATE**
SPITZE UND VORRICHTUNG ZUM AUFTRAGEN UND VERTEILEN VON FLÜSSIGKEITS-, GEL- ODER PASTENPRODUKT
TIP AND DEVICE FOR APPLYING AND SPREADING A LIQUID, GEL OR PASTE PRODUCT

(30) Priorité: 29.03.2018 FR 1852712
(43) Date de publication de la demande: 17.02.2021
(73) Titulaire: Primequal SA, 1268 Begnins (CH)
(72) Inventeur: WEILL, David, 1268 Begnins (CH); CHARPILLOZ, Jérémy, 1234 Vessy (CH); PROST-PETIT-JEAN, Philippe, 39130 Bonlieu (FR)
(74) Mandataire: Bugnion Genève
(86) Numéro de dépôt international: PCT/IB2019/052560
(87) Numéro de publication internationale: WO 2019/186465

(56) Documents cités:
- WO-A1-2005/107961
- WO-A1-2005/107961
- US-A- 4 430 075
- US-A- 4 430 075
- US-A- 5 944 698
- US-A- 5 944 698
- US-A1- 2003 015 557
- US-A1- 2003 015 557
- US-A1- 2004 122 377
- US-A1- 2004 122 377
- US-A1- 2005 063 768
- US-A1- 2005 063 768
- US-A1- 2009 108 033
- US-A1- 2009 108 033
- US-A1- 2013 004 230
- US-A1- 2013 004 230
- US-A1- 2014 016 988
- US-A1- 2014 016 988

## Description

La présente invention concerne un embout d'application et d'étalement d'un produit de type liquide, gel ou pâte ainsi qu'un dispositif comprenant cet embout.

Elle vise notamment à permettre l'application et l'étalement selon une ligne régulière et sans à-coup d'un produit de type colle, colle industrielle, colle médicale, pommade, désinfectant, crème, lubrifiant, colorant se présentant sous forme de liquide, pâte, ou gel.

Elle peut par exemple servir à l'application de colle dite chirurgicale dans le domaine de la médecine, notamment pour refermer les bords d'une plaie ou incision en évitant le recours à des points de suture ou agrafes d'application, qui sont peu agréables voire même douloureux à supporter pour le porteur pendant la phase de cicatrisation, ces points de suture ou agrafes devant être ensuite généralement enlevés après ladite phase de cicatrisation.

En effet l'application de colle de type chirurgicale requière une grande précision, l'application doit être effectuée très régulièrement sans à-coups, avec une épaisseur et une largeur contrôlée, préférablement de l'ordre de 4mm de part et d'autre de la plaie, du cordon de colle, de façon à garantir une bonne fermeture des bords de la plaie et une bonne étanchéité non susceptible de laisser entrer des germes qui pourraient être nocifs à une bonne cicatrisation ou provoquer une infection.

Pour appliquer ce type de colle il est connu par exemple par le document WO 2017 010953 d'utiliser un pistolet d'application, ce type de pistolet est de construction compliquée et n'applique la colle que point par point, ce qui ne permet pas d'obtenir la ligne de collage régulière et contrôlée souhaitée.

Il est également connu par le document WO2013 144849 un dispositif d'application de la colle à une certaine distance de l'utilisateur. Afin de compenser l'extrême rigidité du conteneur, ce dispositif prévoit des moyens pour presser avec force sur un tube pour permettre de délivrer de la colle à une certaine distance de l'utilisateur. Il ne prévoit pas l'application de la colle et encore moins de son étalement.

Il est connu par le document US 2007 0147947 A1 un système d'application de colle comportant un conteneur de colle et une sorte de truelle améliorée. Un tel dispositif ne permet aucune maitrise des doses, ni aucune régularité d'application de la colle.

Par le document US 2015 0266049 est également connu un dispositif d'application de colle, dans lequel celle-ci passe au travers d'un embout poreux de type mousse. Un tel dispositif ne permet pas un étalement précis de la colle. De plus, ce genre de dispositif se bouche rapidement puisque la colle passe à travers la mousse sans qu'il soit possible d'y remédier du fait du caractère intrinsèquement collant de la colle. Pour cette raison de tels dispositifs sont peu utilisables en pratique.

Enfin, par le document US 2016 0015373, est connu un dispositif servant à permettre l'étalement de colle en utilisant une brosse. La brosse ne permet pas d'obtenir une surface régulière, avec de plus un risque de perdre des poils et les voir se mélanger avec le produit à déposer ce qui est un problème si c'est une colle, celle pouvant à terme coller les poils entre eux.

Les document US 2014 016988 et US2013 0004230 décrivent des embouts d'application pour dispenser un liquide.

Le but de la présente invention est de remédier à ces inconvénients et de fournir un dispositif d'application et d'étalement régulier et homogène d'un produit de type liquide, gel ou pâte.

Elle vise notamment à permettre l'étalement selon une ligne régulière et sans à-coup d'un produit, par exemple, de type colle se présentant sous forme de liquide, pâte, ou gel.

Elle vise en particulier à permettre un étalement selon une ligne de largeur et d'épaisseur régulières et homogènes.

Ce ou ces buts sont atteints avec l'embout selon l'invention et avec le dispositif d'application et d'étalement d'un produit de type liquide, gel ou pâte sur une surface donnée, selon l'invention, comprenant des moyens de stockage du produit et un embout d'application selon l'invention.
La présente invention vise un embout d'application 20 pour dispositif d'application et d'étalement d'un produit de type liquide, gel ou pâte sur une surface donnée configuré pour permettre de transformer un volume de produit et de l'étaler sur une surface selon un calibrage (C) prédéterminé comportant
- intérieurement, un moyen 25 de régulation de débit,
- intérieurement, au moins un volume tampon,
- des moyens (31) de dispersion contrôlée du produit dans, lequel l'embout (20) comporte également extérieurement deux séries d'ailettes périphériques circulaires (35) et deux paires d'ailettes (27) disposées de manière diamétralement opposée, lesdites séries d'ailettes périphériques circulaires (35) étant disposées sur l'extérieur de la partie d'application (22) de l'embout (20) à proximité de la face d'étalement (30), lesdites paires d'ailettes (27) diamétralement opposées étant disposées sur l'extérieur de la partie de connexion (21) de l'embout (20) ou sur l'extérieur de l'embout (20) à l'intersection de la partie de connexion (21) et de la partie d'application (22).

De préférence, les moyens 31 de dispersion contrôlée du produit sont constitués par au moins une rainure transversale sur une surface à l'extrémité de l'embout d'application.

Avantageusement l'embout selon l'invention comprend des moyens 31 d'uniformisation de la dispersion du produit, de préférence les moyens 31 d'uniformisation de la dispersion du produit consistent en des rainures de dispersion disposées en croix.

La présente invention vise encore un dispositif d'application et d'étalement d'un produit de type liquide, gel ou pâte sur une surface donnée comprenant des moyens 13 de stockage du produit, ledit dispositif comprenant en outre un embout d'application 20 selon la présente invention.

L'embout et le dispositif selon l'invention sont de conception simple. De plus ils sont facilement nettoyables et stérilisables, ce qui est particulièrement avantageux pour des applications de type médicales.

Selon l'invention l'embout d'application comprend deux parties, une partie dite « d'étalement » ou « d'application » du produit et une partie dite « de connexion » ou « de raccordement » au dispositif d'éjection.

Dans le présent texte, les expressions « partie d'étalement » et « partie d'application » visent la même partie ; et les expressions « partie de connexion » et « partie de raccordement » sont utilisées de manière interchangeable.

Avantageusement, l'embout selon l'invention comporte une partie de connexion de forme cylindrique et une partie d'application et d'étalement de forme cylindrique de diamètre plus élevé que celui de la partie de connexion.

De préférence, l'embout d'application selon l'invention est configuré de manière telle que l'axe longitudinal L2 de la partie d'application et d'étalement est incliné par rapport à l'axe longitudinal L1 de la partie de connexion. De manière préférée, l'embout d'application selon l'invention est configuré de manière telle que l'axe longitudinal L2 de la partie d'application et d'étalement est incliné d'un angle α (alpha) allant de 90 à 180°, de préférence de 135° par rapport à l'axe longitudinal L1 de la partie de connexion.

Avantageusement, l'embout selon l'invention comprend des moyens d'étalement du produit selon une surface plane.

Avantageusement, l'embout selon l'invention comprend des moyens de préhension.

Selon l'invention, l'embout d'application comporte intérieurement au moins un volume tampon prévu de façon à conserver un volume 3D prédéterminé pendant le temps nécessaire à la diminution dudit volume de produit dans l'embout au fur et à mesure de l'étalement du produit. Cette disposition garantit qu'il n'y a pas interruption du flux de produit pendant toute l'opération et permet donc de garantir un étalement selon une ligne régulière et sans interruption contrairement aux dispositifs d'application de colle, par exemple chirurgicale, connus jusqu'à présent. Il est donc possible d'appliquer celle-ci selon un cordon continu.

Selon l'invention, l'embout d'application comporte intérieurement au moins un moyen de régulation de débit, le moyen de régulation du débit peut consister en un trou de passage calibré ou un dispositif plus élaboré de type valve de limitation. La régulation de débit est un moyen physique de régulation de l'épaisseur de la surface en deux dimensions (surface 2D) constituant la ligne ou cordon de colle. En déplaçant le dispositif à vitesse régulière, le produit est libéré avec un débit régulier et l'épaisseur et surface de la ligne de collage sera constante et maitrisée.

Selon un mode de réalisation, le volume tampon 23 est disposé en amont du moyen 25 de régulation de débit.

Selon un autre mode de réalisation, le volume tampon 24 est disposé en aval du moyen 25 de régulation de débit. Selon une réalisation particulière le volume tampon 31est disposé à la sortie du moyen 25 de régulation de débit.

L'embout selon l'invention peut comprendre plusieurs volumes tampon situés en amont du moyen 25 de régulation de débit et/ou en aval du moyen 25 de régulation de débit. Avantageusement l'embout selon l'invention comprend un volume tampon 23 disposé en amont du moyen 25 de régulation de débit, un volume tampon 24 disposé en aval du moyen 25 de régulation de débit et un volume tampon 31 disposé à la sortie du moyen 25 de régulation de débit.

Selon l'invention, l'embout d'application comprend des moyens de dispersion contrôlée du produit. En effet puisque l'on souhaite transformer un volume en trois dimensions ou 3D, (la goutte de produit) en une volume en deux dimensions ou 2D (la ligne ou cordon de produit ou colle selon une épaisseur déterminée), il est important de mettre en œuvre des moyens permettant le transport du produit (liquide, pâte, gel) dans une autre direction, à savoir en largeur (c'est-à-dire en direction transversale par rapport à la direction d'application), ces moyens peuvent être constitués par au moins une rainure transversale disposé à l'extrémité de l'embout d'application.

Avantageusement, l'embout selon la présente invention comprend des moyens d'uniformisation de la dispersion du produit. Afin de garantir l'uniformisation de la dispersion indépendamment de la position du dispositif et/ou de l'embout d'étalement, le moyen de dispersion est avantageusement sous la forme de rainures de dispersion disposées par exemple en croix ou tout autre forme adaptée au produit à disperser.

L'embout selon l'invention est avantageusement constitué d'une seule pièce. Il est ainsi particulièrement compact. En outre il comporte de nombreuses parties ayant plusieurs fonctions. Il peut être réalisé par injection plastique et donc à faible coût et peut être réutilisé et/ou nettoyé et/ou stérilisé facilement.

Il est de plus compatible avec des dispositifs d'éjection de produit existant sur le marché.

D'autres caractéristiques et avantages de la présente invention sont mis en évidence à l'aide de la description qui suit d'au moins un exemple d'exécution non limitatif en référence aux dessins schématiques annexés dans lesquels :
- la Figure 1 est une vue en perspective avant du dispositif d'application et d'étalement en cours d'utilisation,
- la Figure 2 est une vue de côté du dispositif de la figure 1, avec l'embout d'application avant emboîtement sur le dispositif d'éjection,
- la Figure 3 est, à échelle agrandie, une vue de côté de l'embout de la figure 2,
- la Figure 4 est une vue en coupe longitudinale de l'embout de la figure 3,
- la Figure 5 est, à échelle agrandie, une vue de détail de la figure 4,
- la Figure 6 est une vue de côté de l'embout en position d'application et étalement,
- la Figure 7 est une vue de face de l'embout de la figure 6,
- la Figure 8 est une vue de l'embout selon la flèche VIII de la figure 3,
- les Figure 9et 10 sont des vues de côté illustrant différentes façons d'utiliser le dispositif d'application selon l'invention,
- la Figure 11 est une vue similaire à la figure 3 montrant le mélange de deux composants dans l'embout,
- la Figure 12 est une vue similaire à la figure 6 selon un autre mode de réalisation.

Dans la description qui suit les éléments du dispositif d'application et d'étalement d'un produit de type liquide, pâte ou gel selon l'invention qui sont connus en soi de l'homme du métier ne seront décrits que de manière simplifiée. Dans la suite on désignera par volume 3D un volume en trois dimensions, par exemple une goutte, et on parlera par contraste de surface 2D, c'est-à-dire de surface en deux dimensions, même si celle-ci présente une certaine épaisseur, considérée alors comme faible à négligeable par rapport au reste de la surface et du volume. Les éléments similaires ou identiques des différents modes de réalisation seront désignés par des références identiques.

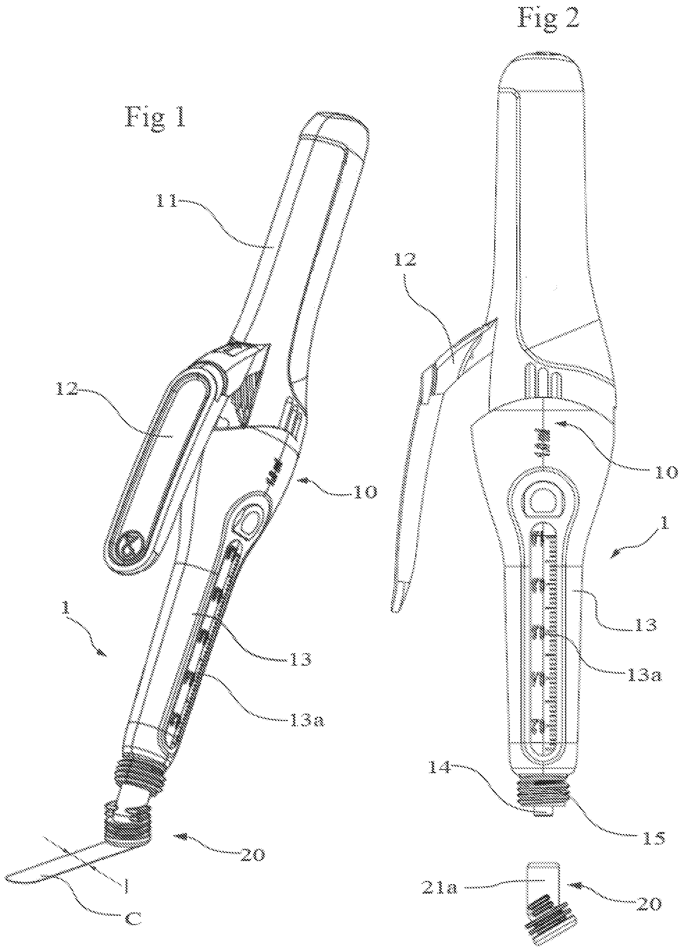

Comme le montrent plus particulièrement les figures 1 et 2, le dispositif d'application et d'étalement 1 d'un produit selon l'invention comporte un dispositif d'éjection 10 de type connu en soi, et un embout 20 d'application et d'étalement pour appliquer et étaler le produit selon une ligne ou cordon C continu et de largeur l régulière.

Le dispositif d'éjection représenté comporte un corps arrière 11, destiné à être pris en main et présentant une forme appropriée à une telle prise en main, il comporte également un réservoir 13 pour le produit devant être appliqué et étalé et, dans le cas représenté des moyens 14, 15 de connexion respectivement par vissage 15 ou par une partie de forme conique 14 à un embout d'application 20 amovible. Le réservoir 13 est muni d'une fenêtre de visualisation graduée 13a. Dans cet exemple, le corps arrière comporte également un levier 12 à cliquet (non représenté) servant à permettre l'éjection du produit. Un tel dispositif déjà connu et décrit par exemple dans le document WO 2008/107813. Bien entendu tout autre type d'éjection est possible en fonction du type de produit à appliquer. Par exemple le système peut être un système à piston, type seringue, ou en cas de produit très fluide on pourra se passer du dispositif à cliquet ou piston, l'application pouvant être effectuée par le seul biais de la gravité. En fonction du type de produit à appliquer et du type de domaine, et notamment dans le domaine médical, il est intéressant que le dispositif d'éjection dispose d'éléments permettant la libération, tel le système à cliquet tel que décrit dans le document WO 2008/107813, d'un dosage régulier et précis du volume de produit qui doit être déposé et étalé.

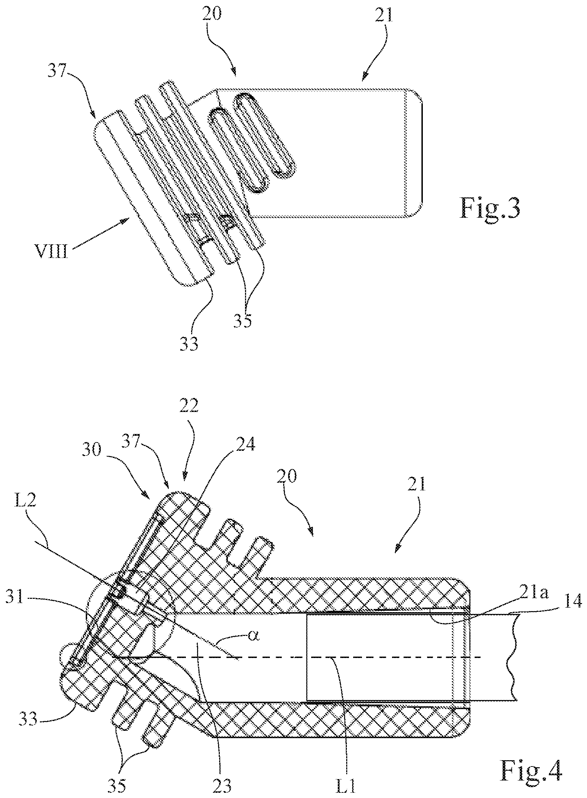

Comme le montre la figure 4, l'embout d'application 20 comporte une partie 21 de raccordement à un dispositif d'éjection et une partie 22 d'étalement du produit délivré par le dispositif d'éjection. La partie de raccordement 21, de forme générale cylindrique, est munie intérieurement d'un évidement cylindrique 21a présentant une forme sensiblement conique complémentaire de celle des moyens de connexion 14 du dispositif d'éjection de façon à pouvoir mettre en place ledit embout d'application 20 sur les moyens de raccordement 14 par coincement.

Dans l'exemple représenté sur la figure 4, les parties coniques de coincement 21 a sont conformes à la norme médicale Luer, on peut aussi envisager des systèmes de raccordement type cône morse ou des systèmes à vis (avec par exemple un pas de vis standard, un pas de vis spécifique, un pas de vis Luer (Luer lock), ou des systèmes de raccordement à verrouillage, par exemple de type baïonnette, clip. On notera que la connexion selon la norme Luer prévue permet un raccordement à tous les dispositifs médicaux connus de type seringue ou système de dosage de précision tel que celui à cliquet évoqué ci-dessus.

L'embout 20 comporte une partie d'étalement 22 de forme généralement cylindrique et de diamètre supérieur à celui de la partie de connexion 21, la partie d'étalement 22 étant destinée plus particulièrement à l'application et étalement du produit. L'axe longitudinal L2 de la partie d'étalement 22 est incliné d'un angle α (alpha) allant de 90 à 180°, de préférence de 135° dans l'exemple représenté, par rapport à l'axe longitudinal L1 de la partie de connexion 21. Cet agencement permet de fournir une bonne ergonomie de l'ensemble ainsi qu'on le verra plus loin. L'extrémité libre de la partie d'étalement 22 définit une face d'étalement 30 qui sera décrite plus en détail plus loin.

La partie d'étalement 22 comprend, intérieurement, un moyen 25 de régulation de débit. En référence au sens d'écoulement du produit dans l'embout, la partie d'étalement 22 comprend en amont du moyen 25 de régulation de débit, un premier évidement 23 créant un volume déterminé. Ce premier évidement 23 est destiné à servir de volume tampon pour le produit devant être appliqué et étalé. Il s'agit en effet de transformer un volume en 3D, par exemple sous forme de goutte, en un volume 2D c'est-à-dire une surface d'étalement « 2D », l'épaisseur de ladite « surface » étant ici considérée comme négligeable par rapport au volume 3D à étaler. Ce volume tampon est prévu de façon à conserver un volume prédéterminé de produit pendant le temps nécessaire à la diminution du volume de produit dans l'embout au fur et à mesure de l'étalement du produit. Au moins un autre évidement 24 ou 31, situé en aval du moyen 25 de régulation de débit peut également être prévu, les évidements 24 et 31 sont généralement de taille plus réduite. L'évidement 31 est prévu dans l'épaisseur de la face d'étalement 30, l'évidement 24 est prévu en amont de l'évidemment 31 dans la partie 22 de l'embout, avantageusement l'évidemment 24 est situé à la sortie du moyen 25 de régulation de débit. Ces évidements sont destinés à servir également de volumes tampons et à être intégrés séparément ou de façon cumulée en fonction des besoins et notamment du type de produit à étaler, de sa viscosité et de la largeur d'étalement souhaitée. L'évidement 24 a une forme sensiblement cylindrique, il est disposé selon l'axe L2 de la partie 22 de l'embout et débouche centralement dans la face d'étalement 30. Selon la figure 4 l'évidement 31 est constitué par deux fentes sensiblement rectangulaires 31 aménagées dans la face d'étalement 30 et disposées à 90° l'une par rapport à l'autre.

Cette disposition garantit qu'il n'y a pas interruption du flux de produit pendant toute l'opération d'application et étalement et permet donc de garantir un étalement selon une ligne régulière et sans interruption, contrairement aux dispositifs d'application de colle chirurgicale connus jusqu'à présent. Il devient donc possible d'appliquer régulièrement et sans à-coups, le produit selon un cordon continu de la largeur et épaisseur souhaitée. Bien entendu les évidements décrits peuvent être choisis avec des formes et/ou volumes différents en fonction du produit à appliquer.

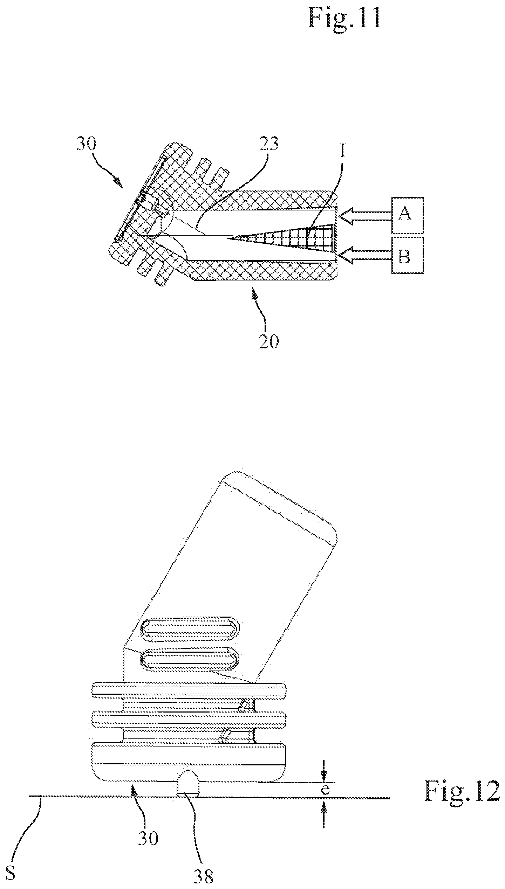

Pour garantir une épaisseur déterminée du cordon de produit appliqué, il peut être prévu d'ajouter par exemple sur la surface d'étalement 30 des formes, par exemple des ergots 38 comme montré dans la figure 12 permettant de garantir une distance/un intervalle déterminé, entre ladite face 30 et la surface d'application à la façon d'une entretoise.

On notera que suivant le type de produit utilisé, par exemple s'il s'agit d'une colle ou autre type de produit sujet à polymérisation, ou à forte capacité d'agglutination, il peut être important de prévoir une zone évitant tout bouchage pendant la délivrance du produit. L'évidement 23 peut dans ce cas être prévu avec une forme géométrique déterminée, par exemple l'évasement important représenté pour éviter l'agglutinement et le bouchage.

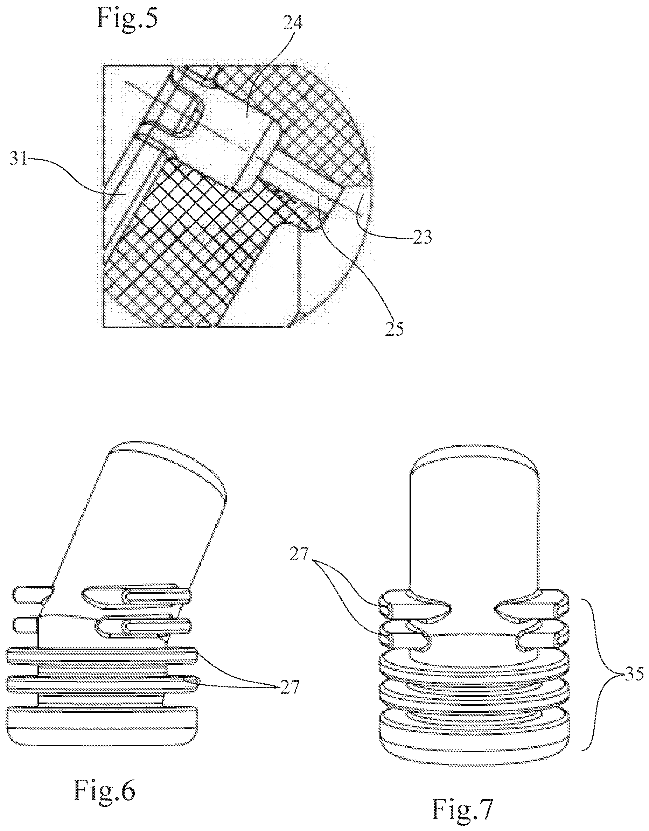

Ainsi que le montre plus particulièrement la figure 5, l'embout d'application 20 comporte également un rétrécissement 25 de forme cylindrique entre l'évidement 23 et l'évidement 24. Le but de ce rétrécissement 25, disposé en amont de la partie d'application/face d'étalement 30 proprement dite est de constituer un moyen de régulation/limitation du débit, afin de limiter physiquement la quantité de produit arrivant sur la face d'étalement et réguler physiquement l'épaisseur de la surface 2D. Ainsi en déplaçant le dispositif 1 à vitesse régulière sur la surface devant être recouverte, le produit est libéré à débit régulier et l'épaisseur de la surface est nécessairement maîtrisée et constante. Un tel rétrécissement peut être remplacé par tout moyen de limitation de débit, tel que par exemple valve de limitation.

Comme indiqué précédemment, la face d'étalement 30 comporte dans l'exemple de réalisation deux fentes sensiblement rectangulaires 31 disposées en croix et communiquant avec l'espace intérieur 24 pour recevoir et permettre l'étalement du produit. Outre la fonction de volume tampon précédemment décrite, ces fentes 31 remplissent également deux autres fonctions. Tout d'abord leur longueur l1 détermine la largeur l du cordon devant être appliqué, ces fentes servent également de guide pour l'écoulement du produit. En fonction de la forme du cordon souhaité et du produit à appliquer, ces fentes 31 pourront avoir une forme différente, être plus larges, plus longues et/ou avoir une longueur différente de la largeur de cordon C souhaité. Par ailleurs la disposition en croix desdites fentes permet de réduire l'influence d'une rotation potentielle du dispositif dans la main de l'utilisateur lors de l'application du produit, tout en assurant une dispersion du produit sur une surface constante et régulière. Bien entendu une autre disposition qu'en croix peut être prévue en fonction du produit et type d'application souhaités. On pourra également avoir un nombre différent de fentes 31. On notera que le débouché de l'évidement 24 qui amène le produit est disposé au centre de la croix formée pas les fentes afin de garantir une répartition régulière du produit.

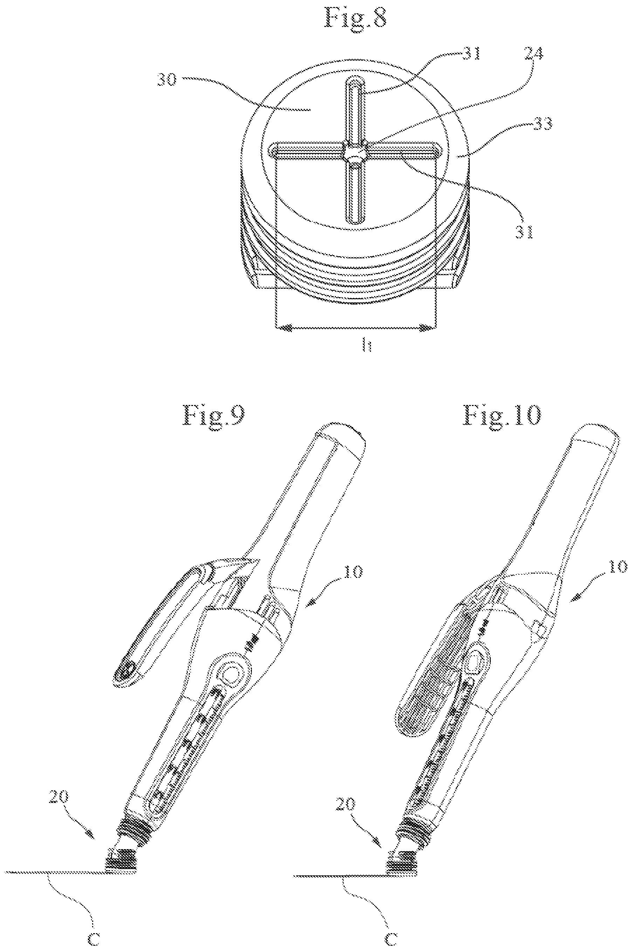

Avantageusement l'embout d'application comprend des moyens d'étalement du produit selon une surface plane 30. Ainsi que le montre la figure 8, la face d'étalement 30 a une forme circulaire et généralement plane, hormis les rainures 31, ce qui permet une face d'appui sur la surface à traiter généralement plane et permet donc un meilleur contact, y compris avec pression lorsque nécessaire sur ladite surface. Comme présenté sur la figure 3, ladite surface d'étalement 30 présente également un chanfrein périphérique 37 arrondi qui facilite le glissement du dispositif sur la surface à traiter et limite les blocages qui peuvent par exemple être inhérents à une application manuelle. Le chanfrein est bien entendu fonction du type de surface, de sa composition, sa régularité. Un chanfrein important peut notamment être intéressant pour glisser plus facilement sur une surface molle ou irrégulière, comme par exemple de la peau.

Le montage par coincement de l'embout 20 dans le dispositif d'application 10 permet un montage selon différentes orientations de l'embout 20 sur ledit dispositif 10, cette fonction est particulièrement intéressante lorsque l'application doit être effectuée manuellement comme dans l'exemple représenté. En effet les différentes possibilités de positionnement permettent de garantir une bonne adaptation à la morphologie de la main et à la gestuelle de l'utilisateur et permettent ainsi de garantir un résultat homogène quel que soit l'utilisateur comme illustré sur les figures 9 et 10.

La figure 11 montre que l'embout selon l'invention peut être utilisé avantageusement avec des produits bi-composants (par exemple colle bi-composant). Dans ce cas les deux composants A et B sont injectés de façon connue en soi par le système mélangeur et le mélange s'effectue au-delà d'un cône I d'injection (qui peut être de forme et de taille variable), au plus proche de la zone 23 de transformation du volume 3D en 2D.

Ainsi que le montrent les figures 3, 6 et 7, l'embout 20 comporte extérieurement deux séries d'ailettes périphériques circulaires 35 et deux paires d'ailettes 27 disposées de manière diamétralement opposée. Les séries d'ailettes périphériques circulaires 35 sont disposées sur l'extérieur de la partie d'application 22 de l'embout 20 à proximité de la face d'étalement 30. Les paires d'ailettes 27 diamétralement opposées sont disposées sur l'extérieur de la partie de connexion 21 de l'embout 20 ou sur l'extérieur de l'embout 20 à l'intersection de la partie de connexion 21 et de la partie d'application 22. Ces ailettes 27, 35 jouent plusieurs fonctions, tout d'abord faciliter la préhension de l'embout 20, en effet cet embout n'est pas toujours intégré au dispositif d'application et il est donc intéressant dans ce cas d'ajouter des éléments améliorants la tenue en main et permettant d'agir avec la force suffisante pour fixer l'embout au dispositif. Ces ailettes peuvent également permettre d'échanger la chaleur en cas de réaction endothermique ou exothermique du produit à délivrer, soit dû au frottement soit à une réaction physique/chimique.

Ainsi que le montre particulièrement la figure 1, la forme de l'embout 20 et plus spécifiquement son angulation permet de garder une bonne vision de la surface de cordon C étalée lors de l'application du produit.

L'utilisation de l'embout avec un dispositif d'application tel que décrit dans le document WO 2008/107813 est particulièrement intéressante puisque ce dispositif délivre des doses contrôlées de produit. Ainsi dans l'exemple de colle chirurgicale, il a été possible de transformer avec succès 20 microlitres de colle en une bande de 4cm de long par 8mm de large.

Bien entendu, la présente invention n'est pas limitée aux modes de réalisation décrits. Bien que décrite en liaison avec des dispositifs médicaux d'application de colle chirurgicale, elle peut être utilisée pour l'application continue et régulière de tout autre type de produit et notamment colle, colle industrielle, pommade, désinfectant, crème, lubrifiant, colorants, peinture, maquillage, gel alcooliques, produits anesthésiants, produit de jointage/étanchéité, sans sortir pour autant du cadre de la présente invention.

## Revendications

1. Embout d'application (20) pour dispositif d'application et d'étalement d'un produit de type liquide, gel ou pâte sur une surface donnée étant configuré pour permettre de transformer un volume de produit et de l'étaler sur une surface selon un calibrage (C) prédéterminé comportant
- intérieurement, un moyen (25) de régulation de débit,
- intérieurement, au moins un volume tampon,
- des moyens (31) de dispersion contrôlée du produit,
**caractérisé en ce que** l'embout (20) comporte également extérieurement deux séries d'ailettes périphériques circulaires (35) et deux paires d'ailettes (27) disposées de manière diamétralement opposée, lesdites séries d'ailettes périphériques circulaires (35) étant disposées sur l'extérieur de la partie d'application (22) de l'embout (20) à proximité de la face d'étalement (30), lesdites paires d'ailettes (27) diamétralement opposées étant disposées sur l'extérieur de la partie de connexion (21) de l'embout (20) ou sur l'extérieur de l'embout (20) à l'intersection de la partie de connexion (21) et de la partie d'application (22).

2. Embout selon la revendication 1 **caractérisé en ce que** les moyens (31) de dispersion contrôlée du produit sont constitués par au moins une rainure transversale sur une surface à l'extrémité de l'embout d'application.

3. Embout selon la revendication 1 ou 2 **caractérisé en ce qu'**il comprend des moyens (31) d'uniformisation de la dispersion du produit.

4. Embout selon la revendication précédente, **caractérisé en ce que** les moyens [31] d'uniformisation de la dispersion du produit consistent en des rainures de dispersion disposées en croix.

5. Embout selon l'une quelconque des revendications précédentes, **caractérisé en ce que** le volume tampon (23) est disposé en amont du moyen (25) de régulation de débit.

6. Embout selon l'une quelconque des revendications précédentes, **caractérisé en ce que** le volume tampon (24) est disposé en aval du moyen (25) de régulation de débit.

7. Embout selon l'une des revendications précédentes **caractérisé en ce qu'**il comporte une partie de connexion de forme cylindrique et une partie d'application et d'étalement de forme cylindrique de diamètre plus élevé que celui de la partie de connexion.

8. Embout selon la revendication précédente, **caractérisé en ce que** l'axe longitudinal L2 de la partie d'application et d'étalement est incliné par rapport à l'axe longitudinal L1 de la partie de connexion.

9. Embout selon la revendication précédente **caractérisé en ce que** l'axe longitudinal L2 de la partie d'application et d'étalement est incliné d'un angle α allant de 90 à 180°, de préférence de 135° par rapport à l'axe longitudinal L1 de la partie de connexion.

10. Embout selon l'une des revendications précédentes **caractérisé en ce qu'**il comprend des moyens d'étalement du produit selon une surface plane (30).

11. Embout selon l'une des revendications précédentes, **caractérisé en ce qu'**il comprend des moyens de préhension (27,35).

12. Embout selon l'une des revendications précédentes, **caractérisé en ce qu'**il est constitué d'une seule pièce.

13. Dispositif d'application et d'étalement d'un produit de type liquide, gel ou pâte sur une surface donnée comprenant des moyens (13) de stockage du produit **caractérisé en ce qu'**il comprend un embout d'application (20) selon l'une quelconque des revendications 1 à 12.

## Patentansprüche

1. Applikationsaufsatz (20) für eine Vorrichtung zum Auftragen und Verteilen eines Produkts vom Typ Flüssigkeit, Gel oder Paste auf eine gegebene Oberfläche, der so konfiguriert ist, dass er ein Produktvolumen umwandeln und es auf einer Oberfläche gemäß einer vorbestimmten Kalibrierung (C) verteilen kann, umfassend
- im Inneren ein Durchflussregelmittel (25),
- im Inneren mindestens ein Puffervolumen,
- Mittel (31) zur kontrollierten Ausbreitung des Produkts,
**dadurch gekennzeichnet, dass**
der Aufsatz (20) außerdem außen zwei Reihen kreisförmiger peripherer Lamellen (35) und zwei Lamellenpaare (27) aufweist, die diametral gegenüberliegend angeordnet sind, wobei die Reihen kreisförmiger peripherer Lamellen (35) an der Außenseite des Applikationsteils (22) des Aufsatzes (20) in der Nähe der Verteilfläche (30) angeordnet sind, wobei die diametral gegenüberliegenden Lamellenpaare (27) an der Außenseite des Verbindungsteils (21) des Aufsatzes (20) oder an der Außenseite des Aufsatzes (20) an der Schnittstelle zwischen dem Verbindungsteil (21) und dem Applikationsteil (22) angeordnet sind.

2. Aufsatz nach Anspruch 1, **dadurch gekennzeichnet, dass** die Mittel (31) zur kontrollierten Ausbreitung des Produkts von mindestens einer Quernut auf einer Oberfläche am Ende des Applikationsaufsatzes gebildet sind.

3. Aufsatz nach Anspruch 1 oder 2, **dadurch gekennzeichnet, dass** er Mittel (31) zur Vereinheitlichung der Produktausbreitung umfasst.

4. Aufsatz nach vorhergehendem Anspruch, **dadurch gekennzeichnet, dass** die Mittel (31) zur Vereinheitlichung der Produktausbreitung aus kreuzförmig angeordneten Verteilnuten bestehen.

5. Aufsatz nach einem der vorhergehenden Ansprüche, **dadurch gekennzeichnet, dass** das Puffervolumen (23) stromaufwärts des Durchflussregelmittels (25) angeordnet ist.

6. Aufsatz nach einem der vorhergehenden Ansprüche, **dadurch gekennzeichnet, dass** das Puffervolumen (24) stromabwärts des Durchflussregelmittels (25) angeordnet ist.

7. Aufsatz nach einem der vorhergehenden Ansprüche, **dadurch gekennzeichnet, dass** er einen zylindrisch geformten Verbindungsteil und einen zylindrisch geformten Applikations- und Verteilteil mit einem größeren Durchmesser als der Verbindungsteil aufweist.

8. Aufsatz nach vorhergehendem Anspruch, **dadurch gekennzeichnet, dass** die Längsachse L2 des Applikations- und Verteilteils in Bezug auf die Längsachse L1 des Verbindungsteils geneigt ist.

9. Aufsatz nach vorhergehendem Anspruch, **dadurch gekennzeichnet, dass** die Längsachse L2 des Applikations- und Verteilteils in einem Winkel α von 90 bis 180°, vorzugsweise 135°, in Bezug auf die Längsachse L1 des Verbindungsteils geneigt ist.

10. Aufsatz nach einem der vorhergehenden Ansprüche, **dadurch gekennzeichnet, dass** er Mittel zum Verteilen des Produkts gemäß einer ebenen Fläche (30) umfasst.

11. Aufsatz nach einem der vorhergehenden Ansprüche, **dadurch gekennzeichnet, dass** er Greifmittel (27, 35) umfasst.

12. Aufsatz nach einem der vorhergehenden Ansprüche, **dadurch gekennzeichnet, dass** er einstückig ausgebildet ist.

13. Vorrichtung zum Auftragen und Verteilen eines Produkts vom Typ Flüssigkeit, Gel oder Paste auf eine gegebene Oberfläche, umfassend Mittel (13) zum Speichern des Produkts, **dadurch gekennzeichnet, dass** sie einen Applikationsaufsatz (20) nach einem der Ansprüche 1 bis 12 umfasst.

## Claims

1. Application tip (20) for a device for applying and spreading a liquid, gel or paste type product on a given surface configured for allowing a volume of product to be transformed and spread on a surface according to a predetermined calibration (C) comprising
- internally, a means (25) for regulating the flow,
- internally, at least one buffer volume,
- means (31) for controlled dispersion of the product.
Chararacterized in that
the tip (20) also comprises externally two series of circular peripheral fins (35) and two pairs of fins (27) arranged diametrically opposite, said series of circular peripheral fins (35) being arranged on the outside of the application part (22) of the tip (20) near the spreading surface (30), said diametrically opposed pairs of fins (27) being arranged on the outside of the connection part (21) of the tip (20) or on the outside of the tip (20) at the intersection of the connection part (21) and the application part (22).

2. Tip according to claim 1 **characterized in that** the means (31) for controlled dispersion of the product consist of at least one transverse groove on a surface at the end of the application tip.

3. Tip according to claim 1 or 2, **characterized in that** it comprises means (31) for homogenizing the dispersion of the product.

4. Tip according to the preceding claim, **characterized in that** the means [31] for homogenizing the dispersion of the product consist of dispersion grooves arranged in the form of a cross.

5. Tip according to any one of the preceding claims, **characterized in that** the buffer volume (23) is disposed upstream of the means (25) for regulating the flow rate.

6. Tip according to any one of the preceding claims, **characterized in that** the buffer volume (24) is disposed downstream of the means (25) for regulating the flow rate.

7. Tip according to one of the preceding claims, **characterized in that** it comprises a cylindrical shaped connection part and a cylindrical shaped application and spreading part of greater diameter than that of the connection part.

8. Tip according to the preceding claim, **characterized in that** the longitudinal axis L2 of the application and spreading part is inclined relative to the longitudinal axis L1 of the connection part.

9. Tip according to the preceding claim **characterized in that** the longitudinal axis L2 of the application and spreading part is inclined at an angle α covering from 90 to 180°, preferably 135° relative to the longitudinal axis L1 of the connection part.

10. Tip according to one of the preceding claims, **characterized in that** it comprises means for spreading the product along a flat surface (30).

11. Tip according to one of the preceding claims, **characterized in that** it comprises gripping means (27, 35).

12. Tip according to one of the preceding claims, **characterized in that** it is made of a single part.

13. Device for applying and spreading a liquid, gel or paste type product on a given surface comprising means (13) for storing the product, **characterized in that** it comprises an application tip (20) according to any one of claims 1 to 12.
